# EUROPEAN PATENT SPECIFICATION

(11) **EP 1 558 368 B1**
(45) Date of publication and mention of the grant of the patent: **27.09.2006**
(21) Application number: 03757314.4
(22) Date of filing: 03.06.2003
(51) Int. Cl.: B01D 57/02, C07K 1/26

(54) **METHOD FOR PH-BIASED ISOELECTRIC TRAPPING SEPARATIONS**
VERFAHREN ZUR ISOELEKTRISCHEN AUFTRENNUNG MITTELS PH-WERT-VOREINSTELLUNG
PROCEDE DE SEPARATION DE PIEGEAGE ISOELECTRIQUE A PH POLARISE

(30) Priority: 05.06.2002 US 163885
(43) Date of publication of application: 03.08.2005
(73) Proprietor: THE TEXAS A & M UNIVERSITY SYSTEM, College Station, TX 77843-3369 (US)
(72) Inventor: VIGH, Gyula, TX 77843-3369 (US)
(74) Representative: Jump, Timothy John Simon
(86) International application number: PCT/US2003/017300
(87) International publication number: WO 2003/103811

(56) References cited:
- EP-A- 0 369 945
- US-A- 4 243 507
- US-A1- 2002 043 465
- US-A1- 2002 060 154
- RIGHETTI P G ET AL: "Protein purification in multicompartment electrolyzers with isoelectric membranes" JOURNAL OF CHROMATOGRAPHY B: BIOMEDICAL SCIENCES & APPLICATIONS, ELSEVIER SCIENCE PUBLISHERS, NL, vol. 699, no. 1-2, 10 October 1997 (1997-10-10), pages 105-115, XP004094992 ISSN: 1570-0232

## Description

### Technical Field

The field of the present application is the separation and concentration of ampholytic compounds. More specifically, the present application relates to a method for altering the composition of samples that contain at least one ampholytic sample component employing pH-biased isoelectric trapping techniques.

### Background

Electrophoretic techniques, and isoelectric focusing (IEF) techniques in particular, remain key technologies for the separation of ampholytic components, small and large, simple and complex alike. Used in many fields and industries, IEF is performed both on an analytical and preparative scale. For example, IEF is utilized in clinical diagnosis, biotechnology, pharmaceutical and food industries, etc., alone or coupled with other analytical or preparative techniques.

In IEF, ampholytic components are separated with the help of an electric field in a pH gradient wherein the pH increases from a lower pH value at the anode to a higher pH value at the cathode. (For a monograph on IEF, see, e.g., P.G. Righetti, Isoelectric focusing: theory, methodology and applications, Elsevier Biomedical, Amsterdam, 1983). Since the net charge of an ampholytic component is zero in its isoelectric state, the electrophoretic migration velocity of an ampholytic component becomes zero whenever the pH of its environment becomes equal to its isoelectric point (pl) value. Thus, ampholytic components with different pl values stop migrating at different points in the pH gradient.

Relatively stable continuous pH gradients can be created by several means. For example, mixtures of carrier ampholytes (compounds that have adequate buffering ability and conductivity in the vicinity of their pl value) may be used. Also, appropriate amounts of suitable weak acids and weak bases or weak acids and strong bases or strong acids and weak bases may be bound, in a spatially controlled manner, into an ion-permeable matrix, such as a cross-linked polyacrylamide gel to preform and stabilize the pH gradient which is then used for immobilized pH gradient IEF (IPGIEF). (For a monograph on IPGIEF, see, e.g., P.G. Righetti, Immobilized pH gradients: theory and methodology, Elsevier, Amsterdam, 1990).

Alternatively, ampholytic sample components can also be separated from each other by isoelectric trapping (IET) utilizing isoelectric membrane-based multicompartmental electrolyzers (e.g., Faupel et al., U.S. Patent No. 5,082,548) wherein at the end of an IET separation process, ampholytic sample components are obtained in their isoelectric state.

### Summary of Invention

Embodiments of the present application electrophoretically alter the initial composition of a sample that contains at least one ampholytic component using a pH-biased isoelectric trapping (IET) process by adding one or more suitable isoelectric buffers that have adequate buffering capacity, titrating capacity, and conductivity in the vicinity of their pl values to obtain an ampholytic sample component in its non-isoelectric state at the end of the IET process.

Briefly, a method practiced according to the present application provides for selecting an electrophoretic separation system having at least one ion-permeable isoelectric barrier, selecting an anolyte having a pH value lower than the pl value of the ampholytic sample component, selecting a catholyte having a pH value higher than the pl value of the ampholytic sample component, providing an isoelectric buffer having a pl value higher than the pH value of the anolyte and lower than the pH value of the catholyte and different from the pl value of the ampholytic sample component, introducing the sample and the isoelectric buffer into the electrophoresis system and trapping the ampholytic sample component in a non-isoelectric state by executing an electrophoretic separation.

One aspect provides for selecting an electrophoretic separation system having one ore more separation compartments. Another aspect provides for selecting two or more an ion-permeable isoelectric barriers having pl values different from the pl values of the ampholytic sample component and the isoelectric buffer.

Another aspect provides for selecting a second isoelectric buffer having a pl value different from the pl values of the ampholytic sample component, the first isoelectric buffer, and the isoelectric barrier. Another aspect provides for trapping a first ampholytic sample component and a second ampholytic sample component in their non-isoelectric states on either side of an isoelectric barrier by executing an electrophoretic separation.

In a first aspect, the present invention provides a method for separating an ampholytic component by electrophoresis, the method comprising:
placing a sample containing an ampholytic component having a pl value in an electrophoresis separation system comprising an anolyte having a pH and a catholyte having a pH, the catholyte pH being higher than the anolyte pH, one or more ion-permeable barriers disposed between the anolyte and catholyte wherein at least one of the barriers is anisoelectric barrier having a pl value which is higher than the anolyte pH and lower than the catholyte pH;
providing an isoelectric buffer having a pl value higher than the pH of the anolyte and lower than the pH of the catholyte and different from the pl value of the ampholytic sample component and different from the pl value of an ion-permeable isoelectric barrier; and
exposing the sample to an electric potential so as to trap the ampholytic sample component in a non-isoelectric state in the presence of the isoelectric buffer in the electrophoresis system.

Preferably, the electrophoretic separation system comprises an anode compartment containing an anode and adapted to receive the anolyte, a cathode compartment containing a cathode and adapted to receive the catholyte, and one ion-permeable barrier in the form of an ion-permeable isoelectric barrier having a pl value disposed between the anode compartment and the cathode compartment.

Preferably, the electrophoretic separation system further comprises first and second ion-permeable barriers disposed between the anode compartment and the cathode compartment forming a first separation compartment between the anode compartment and cathode compartment.

In a preferred form, the first and second ion-permeable barriers are isoelectric barriers each having a defined but different pl.

The sample can be provided to at least one of the sample compartment, anode compartment or the cathode compartment. In one preferred form, the sample is provided to a sample compartment and the ampholytic component is separated in a non-isoelectric state in the sample compartment. Alternatively, the ampholytic component can be obtained in a non-isoelectric state in the sample compartment or the cathode compartment or the anode compartment.

It will be appreciated that the sample can contain two or more ampholytic components which are obtained in non-isoelectric states in the sample compartment or the cathode compartment or the anode compartment or two or more of the sample compartment, the anode compartment or the cathode compartment.

The electrophoretic separation system may further comprise a third ion-permeable barrier disposed between the anode compartment and the cathode compartment forming a second separation compartment adjacent the first separation compartment, the system having first ion-permeable isoelectric barrier having a first pl value and a second ion-permeable isoelectric barrier having a second pl value different from the first pl value of the first ion-permeable isoelectric barrier.

Preferably, all theion-permeable barriers are isoelectric barriers each having a defined but different pl. It will be appreciated that any one or more of the barriers can be isoelectric barriers.

In this form, the sample can be provided to the first sample compartment, the second sample compartment, both the first and second sample compartments, or the anode compartment or the cathode compartment. The ampholytic component can be obtained in a non-isoelectric state in the first sample compartment or in the second sample compartment, or in the anode compartment, or in the cathode compartment.

The sample can contain two or more ampholytic components which are obtained in a non-isoelectric state in the first sample compartment or in the second sample compartment or the anode compartment or the cathode compartment, or in each of the first sample compartment and second sample compartment.

In another preferred form, the electrophoretic separation system comprises a plurality of ion-permeable barriers disposed between the anode compartment and the cathode compartment forming a plurality of separation compartments disposed between the anode and cathode compartments, wherein two or more ion-permeable barriers are ion-permeable isoelectric barriers each having a different pl value. Three or more of the ion-permeable barriers can be ion-permeable isoelectric barriers each having a different pl value. It will be appreciated that all the ion-permeable barriers can be ion-permeable isoelectric barriers each having a different pl value.

The method can include providing a first isoelectric buffer and a second isoelectric buffer each having a different pl value to at least two of the sample compartments or the anode compartment or the cathode compartment.

The ion-permeable barriers can be selected from any suitable material or substance such as immiscible liquids, porous solids, non-ionic membranes, membranes, isoelectric membranes, hydrogel membranes, gels, and hydrogels.

The present inventor has found that hydrogel membranes are preferably polyether sulfone-based, poly(vinyl) alcohol-based or polyacrylamide-based, It will be appreciated that any other type of hydrogel membrane can be used for the present invention.

Preferably, the non-ionic membranes are supported membranes such as cross-linked polyacrylamide and poly(vinyl) alcohol supported on glass fiber, filter paper, polymeric mesh, and paper. The ion-permeable barriers can be porous frits such as glass frits, ceramic frits, and polymeric frits.

In one preferred form, the ion-permeable barriers can substantially restrict the passage of molecules having a size or hydrated ion-radius greater than a predetermined size.

The ion-permeable isoelectric barriers are preferably isoelectric porous solids, isoelectric membranes, and isoelectric gels. More preferably, the ion-permeable isoelectric barriers are isoelectric membranes.

The ion-permeable barriers preferably prevent substantial convective mixing of solutes between the barriers. In use, preferably there is substantially no convective mixing between the compartments.

The isoelectric buffer can be provided to at least one of a sample compartment, the anode compartment and the cathode compartment. Preferably, the pl value of the isoelectric buffer differs by at least about 0.001 pH units from the pl value of the ampholytic sample component The absolute value of the difference between the pl value and the pKa value closest to the pl value of the isoelectric buffer preferably is less than about 1.5.

The isoelectric buffer can be any suitable buffer which will allow an ampholytic component to be obtained in a substantially non-isoelectric state. Suitable buffers include, but not limited to, iminodiacetic acid, N-methylimino diacetic acid, aspartic acid, glutamic acid, glycyl-aspartic acid, m-aminobenzoic acid, histidyl-glycine, histidyl-histidine, histidine, 1,2-diaminopropionic acid, ornithine, lysine, lysil-lysine, and arginine. Preferably, the isoelectric buffer is glutamic acid or lysine. When two isoelectric buffers are used, the present inventor has found that glutamic acid and lysine are particularly suitable.

The method can further include providing a non-ionic solubilizing agent. Preferably, the solubilizing agent is a non-ionic detergent. Examples include, but not limited to, TWEEN^{™} 20, Brij^{™}30, TRITON^{™} X-100, or NDSB-195.

The method is particularly suitable for the separation of ampholytic sample components such as proteins, polypeptides, oligopeptides, amino phenols, amino phosphonic acids, and amino acids. Preferably, the ampholytic sample component is a protein.

The present invention overcomes problems with prior art isoelectric separations that require the component to be separated in its isoelectric state in that the electrophoretic separation is much faster and there are fewer problems with the solubility of the component.

A further advantage of the present invention is that it can be carried out in any electrophoresis separation system that can be adapted for isoelectric separations. The method does not require a special apparatus and results in the more efficient use of known electrophoresis separation systems.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia before the priority date of each claim of this application.

In order that the present invention may be more clearly understood, preferred forms will be described with reference to the following drawings and examples.

### Brief Description of the Drawings

Figure 1 is a schematic diagram of an isoelectric trapping (IET) unit (Prior Art) (Gradipore, Australia) that may be used to practice embodiments of the present application;
Figure 2 is a schematic diagram indicating the characteristics of representative solutions in the compartments of the IET unit shown in Figure 1 at the beginning of the separation process according to the present application;
Figure 3 is a full-column imaging UV absorbance detector trace of a chicken egg white sample analyzed by capillary isoelectric focusing as described in Example 1;
Figure 4 is a full-column imaging UV absorbance detector trace of aliquots removed at the end of an IET separation from the cathodic and anodic separation compartments of the IET unit that is shown in Figure 1 and was operated as described in Example 2, analyzed by capillary isoelectric focusing as described in Example 1;
Figure 5 is a full-column imaging UV absorbance detector trace of aliquots removed at the end of the IET separation from the cathodic and anodic separation compartments of the IET unit that is shown in Figure 1 and was operated as described in Example 3, analyzed by capillary isoelectric focusing as described in Example 1;
Figure 6 is a full-column imaging UV absorbance detector trace of the aliquot removed from the cathodic and anodic separation compartments of the IET unit that is shown in Figure 1 and was operated as described in Example 4, at the end of the IET separation of the sample obtained from the anodic separation compartment in Example 3, analyzed by capillary isoelectric focusing as described in Example 1;
Figure 7 is a full-column imaging UV absorbance detector trace of aliquots removed at the end of the IET separation from the cathodic and anodic separation compartments of the IET unit that is shown in Figure 1 and was operated as described in Example 5, analyzed by capillary isoelectric focusing as described in Example 1.
Figure 8 is a UV absorbance detector trace recorded during a pressure mediated capillary electrophoretic separation of aliquots removed at the beginning and end of the IET separation from the separation compartment of the IET unit that was operated as described in Example 6.
Figure 9 is a UV absorbance detector trace recorded during a pressure mediated capillary electrophoretic separation of aliquots removed at the beginning and end of the IET separation from the separation compartment of the IET unit that was operated as described in Example 7.
Figure 10 is a full-column imaging UV absorbance detector trace of aliquots removed at the end of the IET separation after eight passes from the cathodic and anodic separation compartments of the IET unit that is shown in Figure 1 and was operated as described in Example 8, analyzed by capillary isoelectric focusing as described in Example 1;

### Mode(s) for Carrying Out the Invention

Embodiments for altering the initial composition of a sample mixture having an ampholytic sample component according to the present application are described in non-limiting detail below.

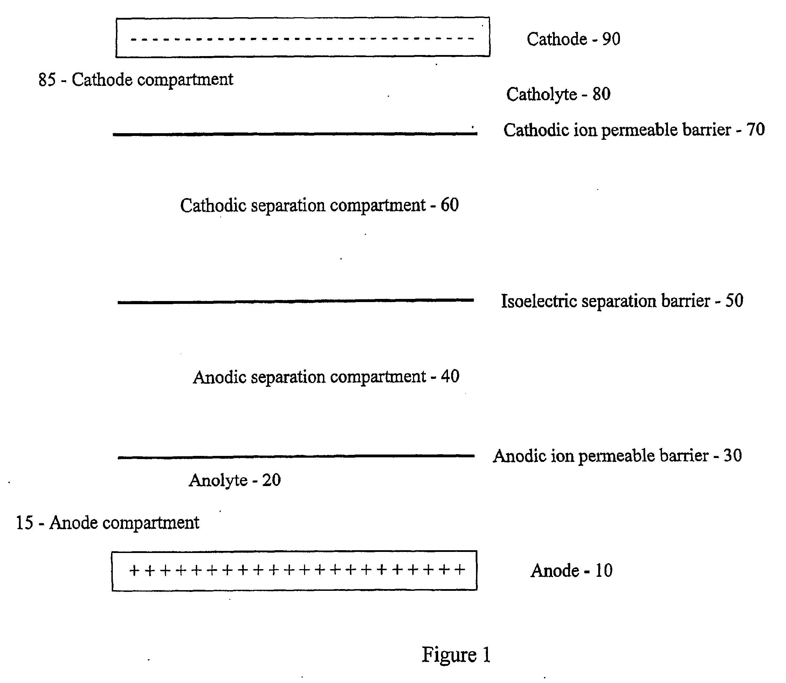

Figure 1 refers to an isoelectric trapping (IET) unit known in the art that may be used to practice embodiments described in the application. In this IET separation unit (Gradipore, Australia), anode 10 is located in anode compartment 15. Anolyte 20 occupies anode compartment 15. Anolyte 20 is an acid solution, for example, it is an aqueous solution of phosphoric acid (H₃PO₄). Of course, other suitable acid solutions known in the art may also be used. Anodic ion-permeable barrier 30 separates anode compartment 15 from adjacent anodic separation compartment 40. lon-permeable barrier 30 allows passage of ions but substantially prevents convective mixing of the contents of anode compartment 15 and anodic separation compartment 40. lon-permeable barrier 30 is also commonly referred to as an ion-permeable, anti-convective barrier or restriction membrane. While in this example anodic ion-permeable barrier 30 is an ion-permeable, anti-convective, non-isoelectric barrier, it may also be an ion-permeable, anti-convective, isoelectric barrier, or any other barrier that allows for passage of ions and prevents convective mixing of the contents of anodic separation compartment 40 and anode compartment 15. Ion-permeable isoelectric separation barrier 50, having adequate buffering and titrating capacity at its pl value, separates anodic separation compartment 40 and cathodic separation compartment 60 and substantially prevents convective mixing of the contents of compartments 40 and 60. Isoelectric separation barrier 50 may be an isoelectric membrane, immobilized isoelectric gel, isoelectric gateway, or any other isoelectric barrier known in the art that has adequate buffering and titrating capacity at its pl value.

Cathodic ion-permeable barrier 70 separates cathodic separation compartment 60 and cathode compartment 85 that contains catholyte 80. Cathodic ion-permeable barrier 70 allows passage of ions, but substantially prevents convective mixing of the contents of cathodic separation compartment 60 and cathode compartment 85. Cathodic ion-permeable barrier 70 is also commonly referred to as an ion-permeable, anti-convective barrier or a restriction membrane. While in this example cathodic ion-permeable barrier 70 is an ion-permeable, anti-convective, non-isoelectric barrier, it may also be an ion-permeable, anti-convective, isoelectric barrier or any other barrier that allows for passage of ions and prevents convective mixing of the contents of cathodic separation compartment 60 and cathode compartment 85. Catholyte 80 is a base solution, for example, it is an aqueous solution of sodium hydroxide (NaOH). Other suitable base solutions known in the art may also be used. Cathode compartment 85 contains cathode 90.

While Figure 1 is merely one example of a suitable IET separation unit, other IET separation unit configurations may also be used. The criteria for selecting the elements of a suitable IET unit according to fundamental IET principles are well known in the art (e.g., Faupel et al., U.S. Patent No. 5,082,548). For example, only one separation compartment between anodic ion-permeable barrier 30 and cathodic ion-permeable barrier 70 may be used or more than two separation compartments between anodic ion-permeable barrier 30 and cathodic ion-permeable barrier 70 may be used. Furthermore, anodic ion-permeable barrier 30 and cathodic ion-permeable barrier 70 may be of similar construction or different. For example, they may be ion-permeable, anti-convective, non-isoelectric barriers or ion-permeable, anti-convective, isoelectric barriers, or any other barriers known in the art such that barriers 30 and 70 are ion-permeable and substantially prevent convective mixing of the contents of the adjacent compartments they separate.

While Figure 1 shows an electrophoresis unit or system having three ion-permeable barriers, the present invention may be performed in a system having only one isoelectric barrier separating the anode compartment for the cathode compartment. In this form, the sample and isoelectric buffer can be provided to one or more of the anode and cathode compartments and the component separated in a non-isoelectric state in either of the anode and cathode compartments.

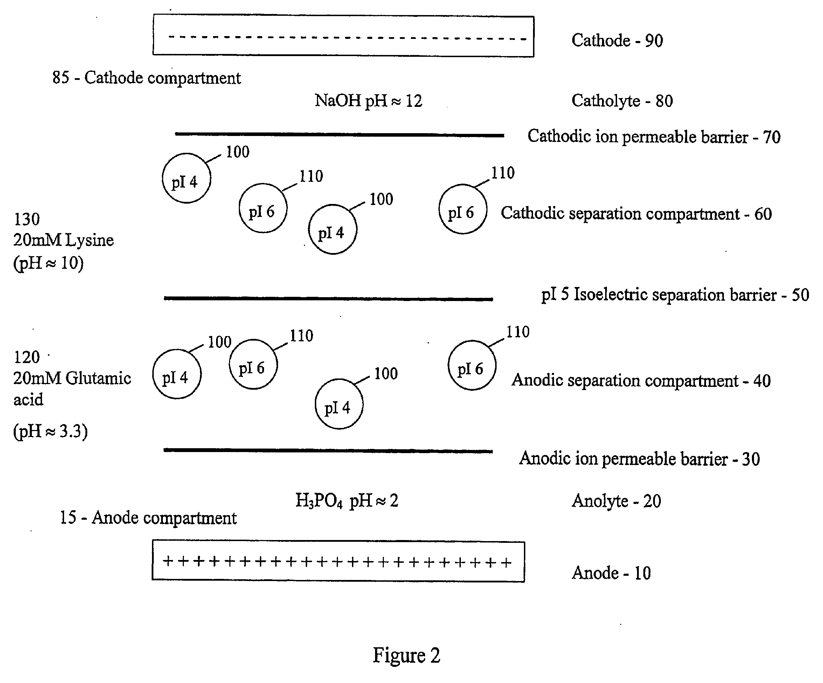

Figure 2 is a schematic diagram indicating the characteristics of representative solutions in the compartments of the IET unit shown in Figure 1 at the beginning of an IET process according to the present application. One feature is the use of an isoelectric buffer in a separation compartment of an IET unit wherein the isoelectric buffer has adequate buffering and titrating capacity and conductivity at its pl value. For example, anodic separation compartment 40 contains a mixture of ampholytic sample components 100 and 110 and first isoelectric buffer 120 wherein first isoelectric buffer has adequate buffering and titrating capacity and conductivity at its pl value, and cathodic separation compartment 60 contains a mixture of ampholytic sample components 100 and 110 and second isoelectric buffer 130 wherein second isoelectric buffer 130 also has adequate buffering capacity, titrating capacity and conductivity at its pl value. The pl value of isoelectric separation barrier 50 is selected to be different from the pl values of first ampholytic sample component 100 and second ampholytic sample component 110. For example, in Figure 2, isoelectric separation barrier 50 is selected to have a pl value between the pl values of ampholytic sample components 100 and 110.

The pl values of first and second isoelectric buffers 120 and 130 are selected to be different both from the pl values of first and second ampholytic sample components 100 and 110, the pl value of isoelectric separation barrier 50, and the pH value of anolyte 20 and catholyte 80 such that at the end of the IET separation process, first and second ampholytic sample components will be present in their non-isoelectric state. In Figure 2, ampholytic sample component 100 is, e.g., a protein with pl = 4, while ampholytic sample component 110 is, e.g., a protein with pl = 6. Isoelectric separation barrier 50 has a pl value of 5, first isoelectric buffer 120 has a pl value of about 3.3, second isoelectric buffer 130 has a pl value of about 10, and anolyte 20 has a pH value of about 2 and catholyte 80 has a pl value of about 12. In this example, isoelectric buffer 120 is a 20 mM glutamic acid solution, isoelectric buffer 130 is a 20mM lysine solution, anolyte 20 is an aqueous solution of phosphoric acid, and catholyte 80 is an aqueous solution of sodium hydroxide.

According to well known IET precepts, application of the electric potential between anode 10 and cathode 90 forces all ampholytic components to migrate into the respective compartments of the IET unit. At the end of the IET separation process, all ampholytic components with pl values higher than the pH value of anolyte 20 and lower than the pl value of isoelectric separation barrier 50 are trapped in anodic separation compartment 40 (i.e., first ampholytic sample component 100 and isoelectric buffer 120 are trapped in anodic separation compartment 40), and all ampholytic components with pl values lower than the pH value of catholyte 80 and higher than the pl value of isoelectric separation barrier 50 are trapped in cathodic separation compartment 60 (i.e., second ampholytic sample component 110 and isoelectric buffer 130 are trapped in cathodic separation compartment 60). However, due to the presence of isoelectric buffer 120 in anodic separation compartment 40, first ampholytic sample component 100 is obtained at the end of the IET process in its non-isoelectric state. Also, due to the presence of isoelectric buffer 130 in cathodic separation compartment 60, second ampholytic sample component 110 is obtained at the end of the IET process in its non-isoelectric state.

Practitioners in the art will appreciate that more than two ampholytic sample components may be present in the sample at any one time. Practitioners in the art will also appreciate that first isoelectric buffer 120 and second isoelectric buffer 130 may be introduced into compartments 40 and/or 60 separate from each other (e.g., isoelectric buffer 120 may be introduced into anodic separation compartment 40 and isoelectric buffer 130 may be introduced into cathodic separation compartment 60), individually mixed with the sample (e.g., isoelectric buffer 120 and a mixture of ampholytic sample components 100 and 110 may be introduced into anodic separation compartment 40, and isoelectric buffer 130 and a mixture of ampholytic sample components 100 and 110 may be introduced into cathodic separation compartment 60), mixed with each other (e.g., isoelectric buffers 120 and 130 may both be introduced into compartments 40 and/or 60), or mixed with each other and the sample (e.g., isoelectric buffer 120, isoelectric buffer 130, ampholytic sample components 100 and 110 may be introduced into compartments 40 and/or 60). Practitioners in the art will further appreciate that there may be more than two ampholytic sample components, more than two separation compartments, more than two isoelectric buffers and more than one isoelectric separation barrier at any one time.

Further, practitioners in the art will appreciate that the sequence or order of introduction of the sample and isoelectric buffer(s) to the separation unit may vary, that is, the sample or buffers may be introduced to the separation unit together or in any sequence.

As a non-limiting example, a mixture of several ampholytic sample components having different pl values may be separated into two parts. In this example, at the end of an IET separation process according to the present application, first ampholytic sample component 100 may comprise a mixture of ampholytic sample components having pl values lower than the pl value of isoelectric separation barrier 50 while second ampholytic sample component 110 may comprise a mixture of ampholytic sample components having pl values higher than the pl value of isoelectric separation barrier 50. Ampholytic sample components 100 and 110 may be any ampholytic sample components that can be separated according to isoelectric focusing or isoelectric trapping principles.

In Figure 2, isoelectric separation barrier 50 is selected to have its pl value higher than the pl value of first ampholytic sample component 100. First isoelectric buffer 120 is selected to have its pl value lower than the pl value of isoelectric separation barrier 50. Although first isoelectric buffer 120 has a pl value lower than isoelectric separation barrier 50, first isoelectric buffer 120 may have a pl value higher or lower than the pl value of first ampholytic sample component 100 because at the end of the IET separation both will force first ampholytic sample component 100 into a non-isoelectric state. For example, in Figure 2, isoelectric buffer 120 has a pl value lower than the pl value of first ampholytic sample 100. However, isoelectric buffer 120 may also have a pl value higher than the pl value of first ampholytic sample component 100.

Similarly, in Figure 2, isoelectric separation barrier 50 is selected to have its pl value lower than the pl value of second ampholytic sample component 110. Second isoelectric buffer 130 is selected to have its pl value higher than the pl value of isoelectric separation barrier 50. Although second isoelectric buffer 130 has a pl value higher than the pl value of isoelectric separation barrier 50, second isoelectric buffer 130 may have a pl value higher or lower than the pl value of second ampholytic sample component 110, because at the end of the IET separation both will force second ampholytic sample component 110 into a non-isoelectric state. For example, in Figure 2, second isoelectric buffer 130 has a pl value higher than the pl value of second ampholytic sample component 110. However, isoelectric buffer 130 may also have a pl value lower than the pl value of second ampholytic sample component 110.

In another embodiment, the pl value of isoelectric buffer 120 is higher than the pl value of isoelectric barrier 50 and the pl value of isoelectric buffer 130 is lower than the pl value of isoelectric barrier 50. Although the pl value of isoelectric buffer 120 is higher than the pl value of isoelectric barrier 50, the pl value of isoelectric buffer 120 is lower than the pl value of second ampholytic sample component 110. Similarly, although the pl value of isoelectric buffer 130 is lower than the pl value of isoelectric separation barrier 50, the pl value of isoelectric buffer 130 is higher than the pl value of first ampholytic sample component 100. In another embodiment, the pl value of isoelectric buffer 120 is higher than the pl value of isoelectric barrier 50 and higher than the pl value of second ampholytic sample component 110, and the pl value of isoelectric buffer 130 is lower than the pl value of isoelectric separation barrier 50 and lower than the pl value of a first ampholytic sample component 100.

While Figure 2 uses two isoelectric buffers, the use of only one isoelectric buffer also practices embodiments of the application. For example, isoelectric buffer 120 may be used without using isoelectric buffer 130. Similarly, isoelectric buffer 130 may be used without using isoelectric buffer 120. For example, the use of a single isoelectric buffer may be advantageous when the sample contains several ampholytic components of dissimilar pl values.

As a non-limiting example of this principle of using only one isoelectric buffer, isoelectric buffer 120 is selected to have a pl value that insures that at the end of the IET separation, isoelectric buffer 120 is present in the same separation compartment as the selected ampholytic sample component (e.g., first ampholytic sample component 100) and renders that sample component non-isoelectric. For example, when first ampholytic sample component 100 has a pl value higher than the pl value of isoelectric separation barrier 50, isoelectric buffer 120 is also selected to have a pl value higher than isoelectric separation barrier 50. Similarly, when first ampholytic sample component 100 has a pl value lower than isoelectric separation barrier 50, isoelectric buffer 120 is selected to have a pl value lower than the pl value of isoelectric separation barrier 50. While isoelectric buffer 120 has a pl value different from the pl value of first ampholytic sample component 100, the pl value of isoelectric buffer 120 may be higher or lower than the pl value of first ampholytic sample component 100. In one embodiment, isoelectric buffer 120 is selected to have a pl value higher than the pl value of first ampholytic sample component 100. In another embodiment, isoelectric buffer 120 is selected to have a pl value lower than the pl value of first ampholytic sample component 100.

In another non-limiting embodiment, isoelectric buffer 120 may be selected to have a pl value that insures that at the end of the IET separation, isoelectric buffer 120 is present in a different separation compartment than first and second ampholytic sample components 100 and 110. In this manner, ampholytic sample components may be obtained on one side of isoelectric separation barrier 50 while non-electrolyte sample components remain on the other side of isoelectric separation barrier 50. For example, in one embodiment, a sample solution contains ampholytic sample components 100 and 110 that have different pl values, along with non-electrolyte sample component 115. The pl value of isoelectric separation barrier 50 is selected such that ampholytic sample components 100 and 110 are located on the same side of isoelectric separation barrier 50 at the end of the IET separation process. For example, in this embodiment, the pl value of isoelectric barrier 50 is selected to be lower than the pl value of both ampholytic sample components 100 and 110. Isoelectric buffer 120 is selected to have a pl value such that at the end of the IET separation process isoelectric buffer 120 is located on the other side of isoelectric separation barrier 50 than ampholytic sample components 100 and 110. For example, in this embodiment, isoelectric buffer 120 is selected to have a pl value lower than both the pl values of ampholytic sample components 100 and 110 and isoelectric separation barrier 50. Cathodic separation compartment 60 is filled with isoelectric buffer 120, while anodic separation compartment 40 may be filled with the sample solution alone, or with a mixture of the sample solution and isoelectric buffer 120. At the end of the IET separation process, anodic separation compartment contains non-electrolyte sample component 115 and isoelectric buffer 120, the latter providing adequate conductivity in anodic separation compartment 40. At the end of the IET separation process, cathodic separation compartment 60 contains a mixture of ampholytic sample components 100 and 110, which provides adequate conductivity in cathodic separation compartment 60.

When the sample is placed only into one of the separation compartments of an IET unit, it may be advantageous, though not necessary, to fill the other separation compartment with isoelectric buffer 120 rather than water, because isoelectric buffer 120 improves the potential distribution across the IET unit and leads to a faster IET separation.

Suitable isoelectric buffers 120 and 130 have adequate buffering and titrating capacity and conductivity in the vicinity of their pl values. In order for an isoelectric buffer to have adequate buffering capacity and adequate conductivity at its pl value, it is preferable that the difference between its pl value and its closest pKa value is less than 1, preferably less than 0.75 and more preferably less than 0.5. For example, suitable isoelectric buffers include N-methylimino diacetic acid, iminodiacetic acid, aspartic acid, glutamic acid, glycyl-aspartic acid, m-aminobenzoic acid, histidyl-glycine, histidyl-histidine, histidine, 1,2-diaminopropionic acid, omithine, lysine, lysil-lysine, and arginine. Practitioners in the art will appreciate that other suitable isoelectric buffers having adequate buffering and titrating capacities and conductivities may also be used.

While Figure 2 illustrates a single binary separation, other embodiments practice sequential binary separations. The isoelectric buffers selected for each sequential IET separation may be the same or different. For example, at the end of a first sequential IET separation, isoelectric buffer 120 may be used to contain both first ampholytic sample components 100 and 110. If the desired target of a second sequential IET separation is first isoelectric sample component 100, and it is desired to be obtained in a solution of isoelectric buffer 135, isoelectric separation barrier 50 for the second sequential separation may be selected to have its pl value between the pl values of first and second isoelectric sample components 100 and 110, isoelectric buffer 120 is selected to have its pl value greater than the pl values of both isoelectric sample components 100 and 110 and isoelectric separation barrier 50. In one embodiment, anodic and cathodic separation compartments 40 and 60 may be filled with a mixture of isoelectric sample components 100 and 110 and isoelectric buffers 120 and 135. In another embodiment, anodic separation compartment 40 may be filled with isoelectric buffer 135 and cathodic separation compartment 60 may be filled with a mixture of isoelectric sample components 100 and 110 and isoelectric buffers 120 and 135. In another embodiment, anodic separation compartment 40 may be filled with isoelectric buffer 135 and cathodic separation compartment 60 may be filled with a mixture of isoelectric sample components 100 and 110 and isoelectric buffer 120. In another embodiment, anodic separation compartment 40 may be filled with isoelectric buffer 135 and cathodic separation compartment 60 may be filled with a mixture of isoelectric sample components 100 and 110 and isoelectric buffers 120 and 135.

In other embodiments, fresh isoelectric buffer may be added in any sequential separation step or isoelectric buffer may be carried over from a previous separation step. Other embodiments practice simultaneous multiple-cut separations (e.g., conducted on an IsoPrime^{™} unit (Amersham-Pharmacia)) with multiple isoelectric buffers.

While the presence of an isoelectric buffer typically enhances the solubility of an ampholytic sample component by rendering it non-isoelectric, suitable solubilizing agents used in IEF systems may also be used in the present application. For example, non-ionic and zwiterrionic solubilizing agents known in the art may also be used to increase solubility of the sample components. For example, TWEEN^{™} 20, Brij^{™} 30, TRITON^{™} X-100 (Aldrich) or any other suitable non-ionic solubilizing agents may also be used. Similarly, NDSB-195^{™} (Toronto Research Chemicals, North York, ON, Canada) or any other suitable zwiterrionic solubilizing agents known in the art may be used.

To assist in understanding the present application, the following examples are included and describe the results of a series of experiments. The following examples relating to this invention should not be construed to specifically limit the invention or such variations of the invention, now known or later developed, which fall within the scope of the invention as described and claimed herein.

### EXAMPLES

### Example 1

### Full-Column UV Absorbance Imaging Capillary Isoelectric Focusing Separation of Chicken Egg White Sample.

Chicken egg white, diluted at a rate of 1 to 25 in deionized water was used as a sample that contains ampholytic components. Full-column UV absorbance imaging capillary isoelectric focusing on an iCE280 instrument (Convergent Bioscience, Toronto, Canada) was used to analyze the chicken egg white sample. The fused silica separation capillary was 5 cm long, its internal diameter was 75 micrometer.

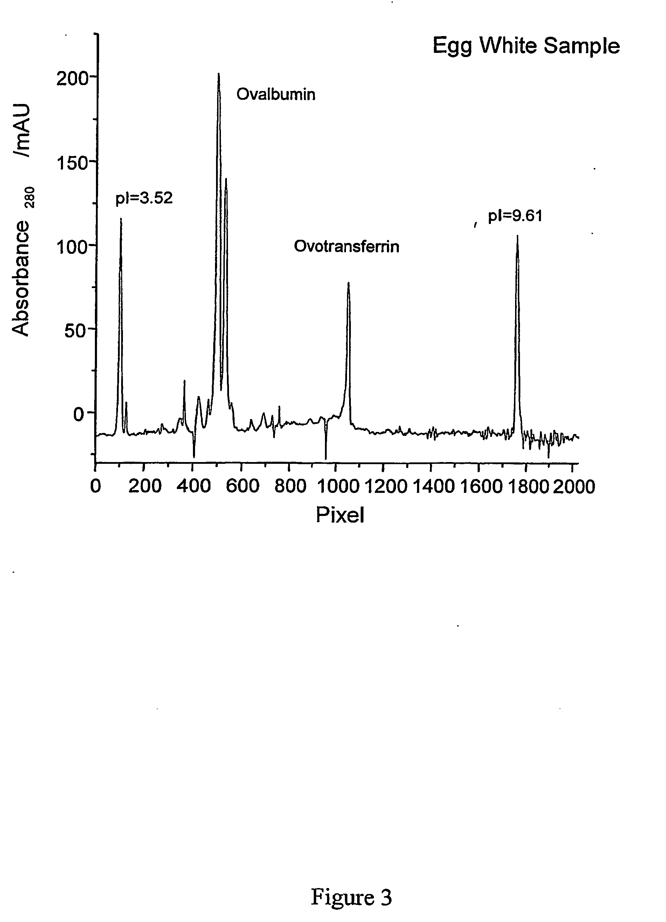

The focusing medium contained 8% carrier ampholytes (Pharmalyte 3-10, Amersham-Pharmacia) covering a pH 3 - 10 range, in an aqueous, 0.28% methylcellulose solution (avg. m.w. = 80,000, Aldrich). 75 microliters of the sample to be analyzed was mixed with 150 microliters of the focusing medium. An aliquot of this solution was filled into the separation capillary and focused for 5 minutes at 3,000 V. Dansyl phenylalanine (approximate pl = 3.52) and terbutaline (approximate pl = 9.61) were used as pl markers. Figure 3 shows the full-column UV absorbance imaging capillary IEF electropherogram of the chicken egg white sample. Ovalbumin focused in a region of the capillary that corresponds to pixels 450 to 650. Ovotransferrin focused in a region of the capillary that corresponds to pixels 1050 to 1150.

### Example 2

### Preparative-Scale Separation of a Chicken Egg White Sample by a Known IET Method (Prior Art).

In order to compare the present application with a known IET method, the preparative-scale separation of a sample that contains ampholytic components was carried out using a known IET method under the conditions described below.

This separation representing the prior art was obtained on an IET unit produced by Gradipore, Australia. The IET cartridge (U.S. Patent No. 6,328,869) contained anodic ion-permeable barrier 30 made of a 5,000 D nominal cut-off polyether sulfone membrane (Gelman), anodic separation compartment 40, pl=4.7 isoelectric separation barrier 50 (Gradipore) prepared from Immobiline^{™} chemicals (Amersham-Pharmacia), acrylamide and N-N'-methylene bis-acrylamide (Amersham-Pharmacia), cathodic separation compartment 60, and cathodic ion-permeable barrier 70 made of a 5,000 D nominal cut-off polyether sulfone membrane (Gelman). 80 mL of a 50 mM phosphoric acid solution (approximate pH = 1.8) was circulated through anode compartment 15 at a flow-rate of 2 Umin. 80 mL of a 50 mM sodium hydroxide solution (approximate pH = 12.4) was circulated through cathode compartment 85 at a flow-rate of 2 Umin. 40 mL each of a filtered chicken egg-white solution (containing egg white diluted with deionized water at a rate of 1 to 25) was circulated through anodic and cathodic separation compartments 40 and 60, respectively, at a flow rate of 20 mL/min. Anode 10 was placed into anode compartment 15, and cathode 90 was placed into cathode compartment 85. The applied potential was initially 250 V, producing a current of 400 mA. After 10 passes of the chicken egg white solution through the separation compartments, the current dropped to 37 mA. The potential was then increased to 500 V, which produced a current of 74 mA. The separation was practically complete after 7 more passes, as indicated by the finishing current leveling off at 25 mA, resulting in a total separation time of 40 minutes. Aliquots were taken from anodic and cathodic separation compartments 40 and 60 after each pass of the chicken egg white solution and analyzed by the full-column UV absorbance imaging capillary IEF instrument as described in Example 1.

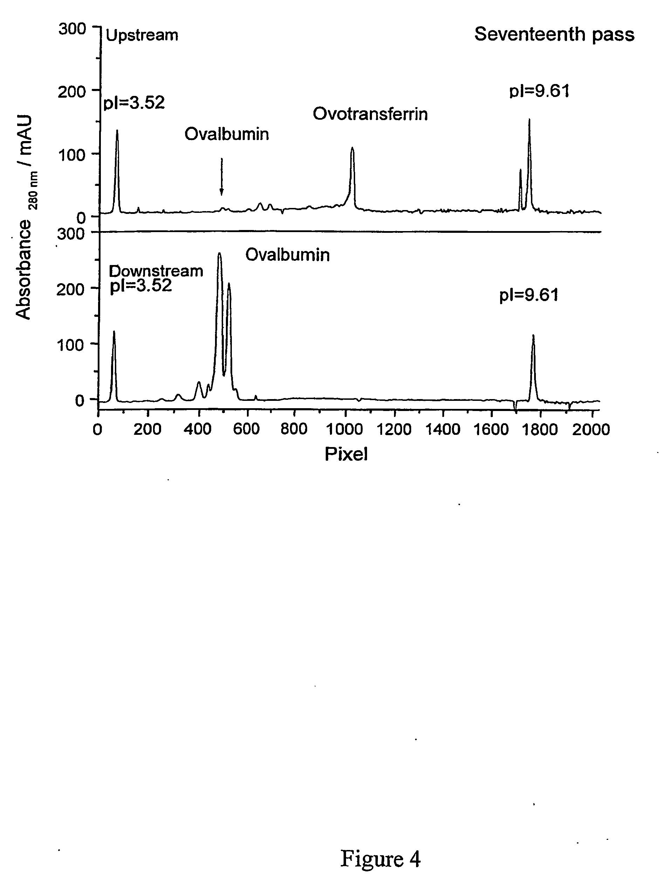

As a result of this IET separation, proteins with pl values lower than 4.7, such as ovalbumin (approximate pl = 4.6), accumulated in anodic separation compartment 40 on the anodic side of pl = 4.7 isoelectric separation barrier 50 (bottom panel in Figure 4, labeled "Downstream"). Proteins with pl values greater than 4.7, such as ovotransferrin (approximate pl = 6.1) accumulated in cathodic separation compartment 60 on the cathodic side of isoelectric separation barrier 50 (top panel in Figure 4, labeled "Upstream"). Practically all of ovalbumin and the lower pl proteins transferred into anodic separation compartment 40. Visual inspection of the interior of the separation cartridge at the end of this IET separation revealed the presence of precipitated protein in anodic separation compartment 40.

### Example 3

### Preparative-Scale Separation of a Chicken Egg White Sample by an IET Separation

The preparative-scale separation of a sample that contains ampholytic components was carried out in the presence of two isoelectric buffers according to the present application under the conditions described below.

The IET separation according to the present application was obtained on the IET unit used in Example 2. The IET cartridge (U.S. Patent No. 6,328,869) contained anodic ion-permeable barrier 30 made of a 5,000 D nominal cut-off polyether sulfone membrane (Gelman), anodic separation compartment 40, pl=4.8 isoelectric separation barrier 50 (Gradipore) prepared from Immobiline^{™} chemicals (Amersham-Pharmacia), acrylamide and N-N'-methylene bis-acrylamide (Amersham-Pharmacia), cathodic separation compartment 60, and cathodic ion-permeable barrier 70 made of a 5,000 D nominal cut-off polyether sulfone membrane (Gelman). 80 mL of a 50 mM phosphoric acid solution (approximate pH = 1.8) was circulated through anode compartment 15 at a flow rate of 2 Umin. 80 mL of a 50 mM sodium hydroxide solution (approximate pH = 12.4) was circulated through cathode compartment 85 at a flow rate of 2 Umin. 40 mL each of a filtered chicken egg white solution (containing chicken egg white dissolved in 20 mM glutamic acid and 20 mM lysine, respectively, at a dilution rate of 1 to 25) was circulated through anodic and cathodic separation compartments 40 and 60, respectively, at a flow rate of 20 mL/min. Anode 10 was placed into anode compartment 15, and cathode 90 was placed into cathode compartment 85. The applied potential was 250 V that initially produced a current of 480 mA. The separation was practically complete after 10 passes of the chicken egg white solution through the separation compartments, indicated by the finishing current leveling off at 53 mA, resulting in a total separation time of 25 minutes. Aliquots were taken from anodic and cathodic separation compartments 40 and 60 after each pass and analyzed by the full-column UV absorbance imaging capillary IEF instrument as described in Example 1.

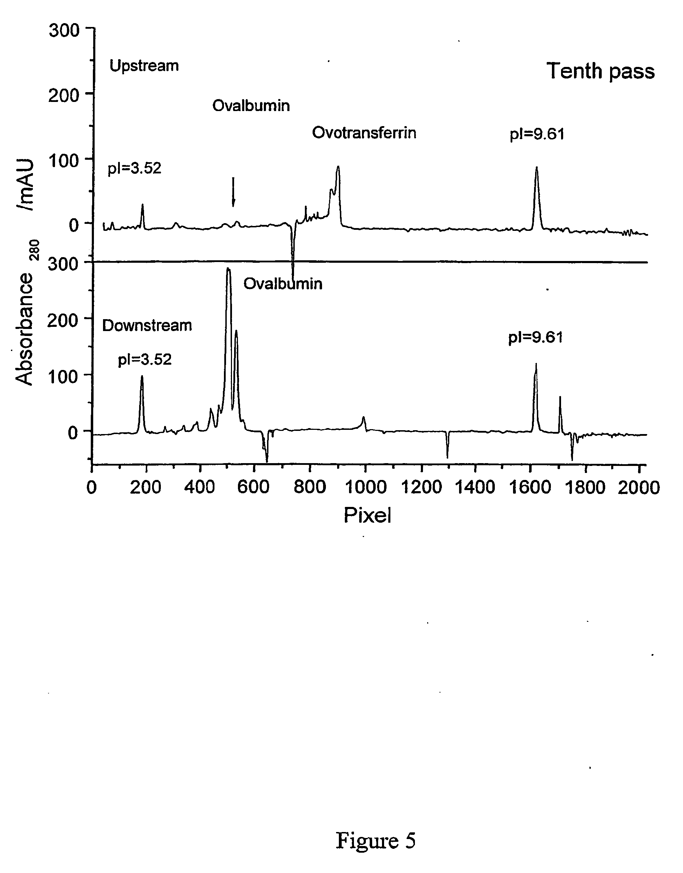

As a result of the IET separation according to the present application, proteins with pl values lower than 4.8, such as ovalbumin (approximate pl = 4.6), accumulated in anodic separation compartment 40 on the anodic side of pl = 4.8 isoelectric separation barrier 50 (bottom panel in Figure 5 labeled "Downstream"). Proteins with pl values greater than 4.8, such as ovotransferrin (approximate pl = 6.1) accumulated in cathodic separation compartment 60 on the cathodic side of isoelectric separation barrier 50 (top panel in Figure 5 labeled "Upstream"). Practically all of ovalbumin and the lower pl proteins transferred into anodic separation compartment 40. Visual inspection of the interior of the separation cartridge at the end of this IET separation indicated that protein precipitation did not occur either in anodic separation compartment 40 or cathodic separation compartment 60.

Thus, effectively the same separation was achieved in Example 3 as in Example 2, but the separation required a shorter period of time and a smaller number of passes of these solutions through the IET cartridge despite the fact that the potential applied over the course of the separation was lower. Additionally, one of the main limitations of known IET separations, i.e., that the low solubility of the ampholytic sample components recovered in their isoelectric state causes their precipitation, has been mitigated.

### Example 4

### Preparative-Scale Sequential Binary Separation of a Chicken Egg White Sample by an IET Separation

The preparative-scale, sequential binary separation of a sample that contains ampholytic components was carried out in the presence of two isoelectric buffers according to the present application under the conditions described below to obtain a fraction from ampholytic components with closely spaced pl values.

In this example, the sample recovered from anodic separation compartment 40 in Example 3 was again subjected to IET separation using an IET cartridge (U.S. Patent No. 6,328,869) that contained anodic ion-permeable barrier 30 made of a 5,000 D nominal cut-off polyether sulfone membrane (Gelman), anodic separation compartment 40, pl=4.5 isoelectric separation barrier 50 (Gradipore) prepared from Immobiline^{™} chemicals (Amersham-Pharmacia), acrylamide and N-N'-methylene bis-acrylamide (Amersham-Pharmacia), cathodic separation compartment 60, and cathodic ion-permeable barrier 70 made of a 5,000 D nominal cut-off polyether sulfone membrane (Gelman). 80 mL of a 50 mM phosphoric acid solution (approximate pH = 1.8) was circulated through anode compartment 15 at a flow rate of 2 Umin. 80 mL of a 50 mM sodium hydroxide solution (approximate pH = 12.4) was circulated through cathode compartment 85 at a flow rate of 2 Umin. 38 mL of the liquid recovered from anodic separation compartment 40 after the IET separation in Example 3 using isoelectric separation barrier 50 with a pl value of 4.8 was circulated through anodic separation compartment 40 at a flow rate of 20 mL/min. Thus, initially, both the pl < 4.8 proteins and the glutamic acid added in Example 3 were present in this stream. 40 mL of a 20 mM lysine solution was circulated through cathodic separation compartment 60 at a flow rate of 20 mL/min. Anode 10 was placed into anode compartment 15, and cathode 90 was placed into cathode compartment 85. The applied potential was 250 V, producing an initial current of 87 mA. After 6 passes of the prefractionated chicken egg white solution, the current leveled off at 68 mA. Aliquots were taken from anodic and cathodic separation compartments 40 and 60 after each pass and analyzed by the full-column UV absorbance imaging capillary IEF instrument as described in Example 1.

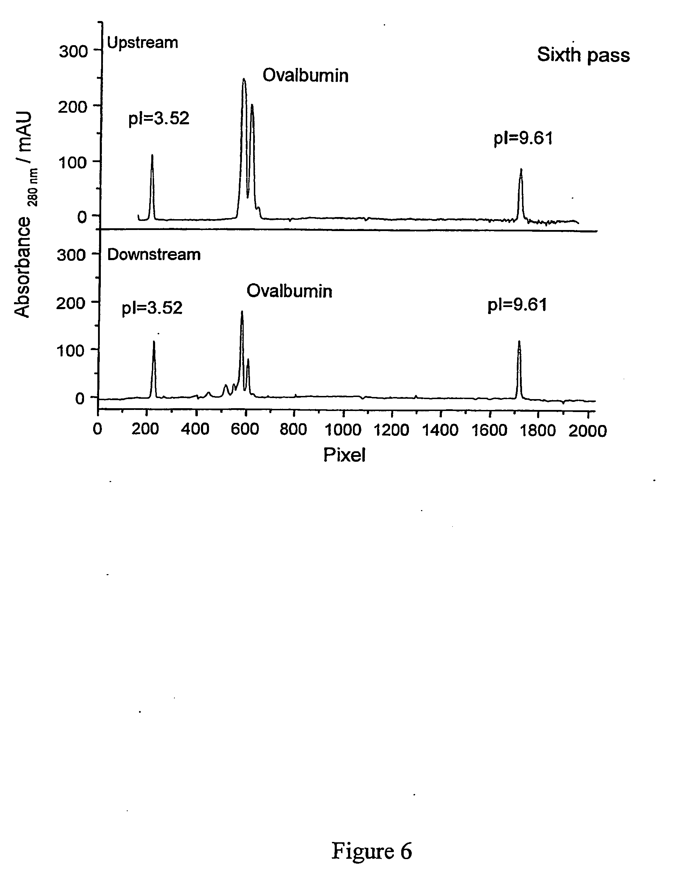

As a result of the sequentially applied second IET separation, proteins with pl values lower than 4.5 remained in anodic separation compartment 40 on the anodic side of pl = 4.5 isoelectric separation barrier 50 (bottom panel in Figure 6 labeled "Downstream"). Proteins with pl values greater than 4.5, such as ovalbumin (approximate pl = 4.6) accumulated in cathodic separation compartment 60 on the cathodic side of isoelectric separation barrier 50 (top panel in Figure 6 labeled "Upstream"). The transfer of ovalbumin was about 90% complete in 6 passes, aided by the presence of glutamic acid in anodic separation compartment 40 and lysine in cathodic separation compartment 60. Notably, cathodic separation compartment 60 did not contain any of pl < 4.5 proteins; these remained in anodic separation compartment 40. Anodic separation compartment 40 contained some left-over pl > 4.5 proteins. Once again, visual inspection of the interior of the IET cartridge at the end of the IET separation according to the present application revealed that there was no protein precipitation in either anodic separation compartment 40 or cathodic separation compartment 60.

### Example 5

### Preparative-Scale Separation of a Chicken Egg White Sample that Contains Ampholytic Components by an IET Separation

The preparative-scale separation of a sample that contains ampholytic components was carried out in the presence of one isoelectric buffer according to the present application under the conditions described below.

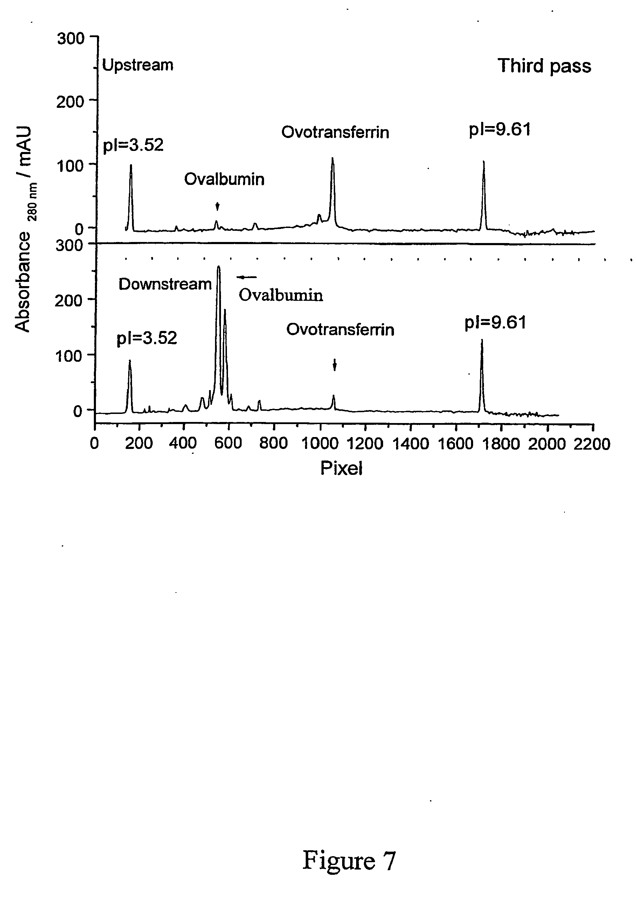

The separation according to the present application was obtained on the IET unit used in Examples 3 and 4using an IET cartridge (U.S. Patent No. 6,328,869) that contained anodic ion-permeable barrier 30 made of a 1,000 D nominal cut-off polyacrylamide membrane (Gradipore), anodic separation compartment 40, pl=5.0 isoelectric separation barrier 50 (Gradipore) prepared from Immobiline^{™} chemicals (Amersham-Pharmacia), acrylamide and N-N'-methylene bis-acrylamide (Amersham-Pharmacia), cathodic separation compartment 60, and cathodic ion-permeable barrier 70 made of a 1,000 D nominal cut-off polyacrylamide membrane (Gradipore). 60 mL of a 2 mM acetic acid solution (approximate pH = 3.9) was circulated through anode compartment 15 at a flow rate of 2 Umin. 60 mL of a 15 mM ethanolamine solution (approximate pH = 10.5) was circulated through cathode compartment 85 at a flow rate of 2 Umin. 30 mL of a filtered chicken egg-white solution (containing chicken egg white diluted at a rate of 1 to 25 in deionized water) was circulated through anodic separation compartment 40 at a flow rate of 20 mL/min. 30 mL of a filtered chicken egg-white solution (containing chicken egg white diluted at a rate of 1 to 25 in 5 mM lysine solution) was circulated through cathodic separation compartment 60 at a flow rate of 20 mL/min. Anode 10 was placed into anode compartment 15, and cathode 90 was placed into cathode compartment 85. The applied potential was 250 V that produced an initial current of 280 mA. Aliquots were taken from anodic and cathodic separation compartments 40 and 60 after each pass and analyzed by the full-column UV absorbance imaging capillary IEF instrument as described in Example 1. The IET separation was almost complete after 3 passes of the chicken egg white solutions, as shown in Figure 7, requiring a total separation time of 6 minutes.

As a result of the IET separation, proteins with pl values lower than 5.0, such as ovalbumin (approximate pl = 4.6), accumulated in anodic separation compartment 40 on the anodic side of pl = 5.0 isoelectric separation barrier 50 (bottom panel in Figure 7 labeled "Downstream"). Proteins with pl values greater than 5.0, such as ovotransferrin (approximate pl = 6.1) accumulated in cathodic separation compartment 60 on the cathodic side of isoelectric separation barrier 50 (top panel in Figure 7 labeled "Upstream"). Practically all of ovalbumin and the lower pl proteins transferred into anodic separation compartment 40.

### Example 6

### Preparative-Scale Desalting of an Ampholytic Sample Mixture by an IET Separation

The preparative-scale desalting of an ampholytic component-containing sample was carried out in the presence of one isoelectric buffer according to the present application under the conditions described below.

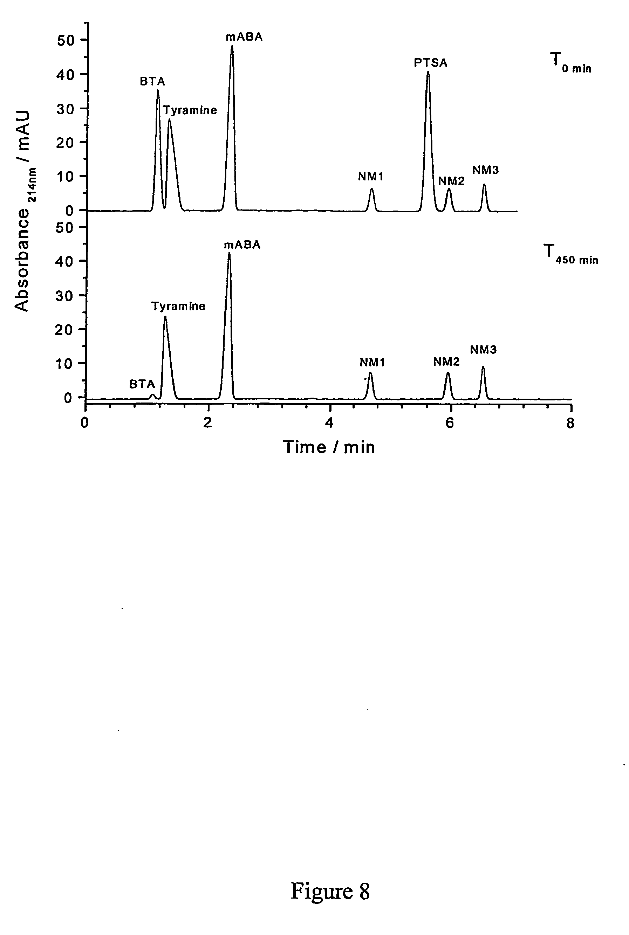

The IET desalting process according to the present application was obtained on the IET unit used in Example 2. The IET cartridge (U.S. Patent No. 6,328,869) contained anodic ion-permeable barrier 30 made of a cross-linked poly(vinyl alcohol) membrane (Gradipore) and cathodic ion-permeable barrier 70 made of a cross-linked poly(vinyl alcohol) (Gradipore) membrane. 200 mL of an 80 mM phosphoric acid solution (approximate pH = 1.6) was circulated through anode compartment 15 at a flow rate of 2 Umin. 200 mL of a 400 mM sodium hydroxide solution (approximate pH = 13.2) was circulated through cathode compartment 85 at a flow rate of 2 Umin. 30 mL of sample solution (initially containing 30 mM tyramine as an ampholytic sample component, 30 mM m-aminobenzoic acid as an isoelectric buffer and 30mM benzyltrimethylammonium para-toluene sulfonate as a strong electrolyte salt) was circulated through the separation compartment, at a flow rate of 20 mL/min. Anode 10 was placed into anode compartment 15, and cathode 90 was placed into cathode compartment 85. Initially, the applied potential was 5 V that produced a current of 500 mA. The recycled solution was electrophoresed for 450 minutes to demonstrate that ampholytic sample component tyramine could be trapped in the IET unit in a non-isoelectric state (mixed with isoelectric buffer m-aminobenzoic acid) for very long periods of time (7.5 hours). At 450 minutes, an applied potential of 80 V generated an electrophoretic current of 300 mA. Aliquots were taken from separation compartment over the course of the experiment and analyzed by pressure mediated capillary electrophoresis (PreMCE) as described in W.A. Williams and G. Vigh, "A Fast, Accurate Mobility Determination Method for Capillary Electrophoresis", *Analytical Chemistry* 68 (1996) 1174. The PreMCE UV absorbance detector trace of the initial sample is shown in the top panel of Figure 8 (labeled T_{0 min}), that of the last aliquot is shown in the bottom panel of Figure 8 (labeled T_{450 min}). In the detector traces, label BTA indicates the peak corresponding to the benzyltrimethylammonium ion, label PTSA indicates the peak corresponding to the para-toluene sulfonate ion, label mABA indicates the peak corresponding to m-aminobenzoic acid, while labels NM1, NM2 and NM3 indicate the peaks corresponding to nitromethane, an internal standard in PreMCE analysis.

As a result of the IET separation according to the present application, strong electrolyte salt benzyltrimethylammonium para-toluene sulfonate was removed from the sample that contained ampholytic component tyramine. Tyramine was trapped in the separation compartment along with m-aminobenzoic acid and was in its non-isoelectric state. Thus, the initial composition of the sample has been successfully altered and the ampholytic sample component was trapped in the IET unit in its non-isoelectric state.

### Example 7

### Preparative-Scale Desalting of a Chicken Egg White Sample by an IET Separation

The preparative-scale desalting of a sample that contains ampholytic components was carried out in the presence of two isoelectric buffers according to the present application under the conditions described below.

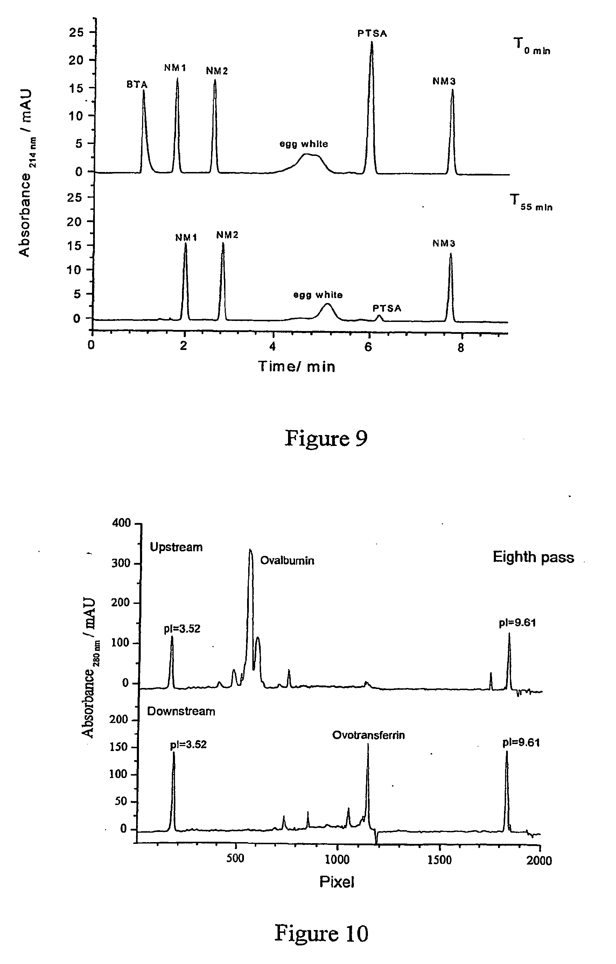

The IET desalting process according to the present application was carried out on the IET unit used in Example 2. The IET cartridge (U.S. Patent No. 6,328,869) contained anodic ion-permeable barrier 30 made of a 5,000 D nominal cut-off polyether sulfone membrane (Gelman) and cathodic ion-permeable barrier 70 made of a 5,000 D nominal cut-off polyether sulfone membrane (Gelman). 200 mL of a 50 mM phosphoric acid solution (approximate pH = 1.8) was circulated through anode compartment 15 at a flow rate of 2 Umin. 200 mL of a 50 mM sodium hydroxide solution (approximate pH = 12.4) was circulated through cathode compartment 85 at a flow rate of 2 Umin. 80 mL of a filtered chicken egg white solution (containing chicken egg white as ampholytic sample components dissolved at a rate of 1 to 25 in a solution that also contained 20 mM glutamic acid as first isoelectric buffer, 20 mM lysine as second isoelectric buffer, and 25mM benzyltrimethylammonium para-toluene sulfonate as strong electrolyte salt) was circulated through the separation compartment at a flow rate of 40 mL/min. Anode 10 was placed into anode compartment 15, and cathode 90 was placed into cathode compartment 85. The initially applied potential was 35 V that produced a current of 500 mA. The bulk of benzyltrimethylammonium para-toluene sulfonate salt was removed from the recirculated chicken egg white solution in as little as 55 minutes, as indicated by the finishing current leveling off at 24 mA. Aliquots were taken from the separation compartment over the course of the experiment and analyzed by pressure mediated capillary electrophoresis (PreMCE) as described in W.A. Williams and G. Vigh, "A Fast, Accurate Mobility Determination Method for Capillary Electrophoresis", *Analytical Chemistry* 68 (1996) 1174. The PreMCE UV absorbance detector trace of the initial sample is shown in the top panel of Figure 9 (labeled T_{0 min}), that of the last aliquot is shown in the bottom panel of Figure 9 (labeled T_{55 min}). In the detector traces, label BTA indicates the peak corresponding to the benzyltrimethylammonium ion, label PTSA indicates the peak corresponding to the para-toluene sulfonate ion, while labels NM1, NM2 and NM3 indicate the peaks corresponding to nitromethane, an internal standard in PreMCE analysis.

As a result of the IET separation according to the present application, the strong electrolyte salt, benzyltrimethylammonium para-toluene sulfonate was removed from the sample, while the ampholytic sample components, the chicken egg white proteins were trapped in the separation compartment in their non-isoelectric state, mixed both with lysine and glutamic acid. Thus, the initial composition of the sample has been successfully altered and the ampholytic sample components were trapped in the IET unit in their non-isoelectric state in the presence of two isoelectric buffers.

### Example 8

### Preparative-Scale Separation of a Chicken Egg White Sample by an IET Separation

The preparative-scale separation of a sample that contains ampholytic components was carried out in the presence of two isoelectric buffers according to the present application under the conditions described below.

The IET separation according to the present application was obtained on the IET unit used in Example 2. The IET cartridge (U.S. Patent No. 6,328,869) contained anodic ion-permeable barrier 30 made of a pl=3.0 isoelectric barrier (Gradipore) prepared from Immobiline^{™} chemicals (Amersham-Pharmacia), acrylamide and N-N'-methylene bis-acrylamide (Amersham-Pharmacia), anodic separation compartment 40, pl=5.0 isoelectric separation barrier 50 (Gradipore) prepared from Immobiline^{™} chemicals (Amersham-Pharmacia), acrylamide and N-N'-methylene bis-acrylamide (Amersham-Pharmacia), cathodic separation compartment 60, and cathodic ion-permeable barrier 70 made of a pl=8.2 isoelectric barrier (Gradipore) prepared from Immobiline^{™} chemicals (Amersham-Pharmacia), acrylamide and N-N'-methylene bis-acrylamide (Amersham-Pharmacia). 80 mL of a 10 mM phosphoric acid solution was circulated through anode compartment 15 at a flow rate of 2 Umin. 80 mL of a 10 mM triethanolamine solution was circulated through cathode compartment 85 at a flow rate of 2 Umin. 30 mL each of a filtered chicken egg white solution (containing chicken egg white dissolved in 20 mM glutamic acid and 20 mM histidine, respectively, at a dilution rate of 1 to 25) was circulated through anodic and cathodic separation compartments 40 and 60, respectively, at a flow rate of 20 mL/min. Anode 10 was placed into anode compartment 15, and cathode 90 was placed into cathode compartment 85. The initial electrophoresis current was set at 250 mA. The separation was practically complete after 8 passes of the chicken egg white solution through the separation compartments, resulting in a total separation time of 16 minutes. Aliquots were taken from anodic and cathodic separation compartments 40 and 60 after each pass and analyzed by the full-column UV absorbance imaging capillary IEF instrument as described in Example 1.

As a result of the IET separation according to the present application, proteins with pl values lower than 5.0, such as ovalbumin (approximate pl = 4.6), accumulated in anodic separation compartment 40 on the anodic side of pl = 5.0 isoelectric separation barrier 50 (bottom panel in FIG. 10 labelled "Downstream"). Proteins with pl values greater than 5.0, such as ovotransferrin (approximate pl = 6.1) accumulated in cathodic separation compartment 60 on the cathodic side of isoelectric separation barrier 50 (top panel in FIG. 10 labelled "Upstream"). Practically all of ovalbumin and the lower pl proteins transferred into anodic separation compartment 40. Visual inspection of the interior of the separation cartridge at the end of this IET separation indicated that protein precipitation did not occur either in anodic separation compartment 40 or cathodic separation compartment 60.

## Claims

1. A method for separating an ampholytic component by electrophoresis, the method comprising:
placing a sample containing an ampholytic component having a pl value in an electrophoresis separation system comprising an anolyte having a pH and a catholyte having a pH, the catholyte pH being higher than the anolyte pH, one or more ion-permeable barriers disposed between the anolyte and catholyte wherein at least one of the barriers is an isoelectric barrier having a pl value which is higher than the anolyte pH and lower than the catholyte pH;
providing an isoelectric buffer having a pl value higher than the pH of the anolyte and lower than the pH of the catholyte and different from the pl value of the ampholytic sample component and different from the pl value of an ion-permeable isoelectric barrier; and
exposing the sample to an electric potential so as to trap the ampholytic sample component in a non-isoelectric state in the presence of the isoelectric buffer in the electrophoresis system.

2. The method according to claim 1 wherein the electrophoretic separation system comprises an anode compartment containing an anode and adapted to receive the anolyte, a cathode compartment containing a cathode and adapted to receive the catholyte, and one ion-permeable barrier in the form of an ion-permeable isoelectric barrier having a pl value disposed between the anode compartment and the cathode compartment.

3. The method according to claim 2 wherein the electrophoretic separation system further comprises first and second ion-permeable barriers disposed between the anode compartment and the cathode compartment forming a first separation compartment between the anode compartment and cathode compartment.

4. The method according to claim 3 wherein the first and second ion-permeable barriers are ion-permeable isoelectric barriers each having a defined but different pl.

5. The method according to claim 3 or 4 wherein the sample is provided to at least one of the sample compartment, anode compartment or the cathode compartment.

6. The method according to claim 5 wherein the sample is provided to the sample compartment and the ampholytic component is separated in a non-isoelectric state in the sample compartment.

7. The method according to claim 5 wherein the ampholytic component is obtained in a non-isoelectric state in the sample compartment or the cathode compartment or the anode compartment.

8. The method according to claim 3 or 4 wherein the sample contains two or more ampholytic components which are obtained in non-isoelectric states in the sample compartment or the cathode compartment or the anode compartment or two or more of the sample compartment, the anode compartment or the cathode compartment.

9. The method according to claim 3 wherein the electrophoretic separation system further comprises another ion-permeable barrier disposed between the anode compartment and the cathode compartment forming a second separation compartment adjacent the first separation compartment, the system having a first ion-permeable isoelectric barrier having a first pl value and a second ion-permeable isoelectric barrier having a second pl value different from the first pl value of the first ion-permeable isoelectric barrier.

10. The method according to claim 9 wherein the ion-permeable barriers are all isoelectric barriers each having a defined but different pl.

11. The method according to claim 9 or 10 wherein the sample is provided to the first sample compartment, the second sample compartment, the anode compartment or the cathode compartment, or both the first and second sample compartments.

12. The method according to claim 11 wherein the ampholytic component is obtained in a non-isoelectric state in the first sample compartment or in the second sample compartment, or in the anode compartment, or the cathode compartment.

13. The method according to claim 9 or 10 wherein the sample contains two or more ampholytic components which are obtained in a non-isoelectric state in the first sample compartment or in the second sample compartment, or in each of the first sample compartment and second sample compartment.

14. The method according to claim 2 wherein the electrophoretic separation system further comprises a plurality of ion-permeable barriers disposed between the anode compartment and the cathode compartment forming a plurality of separation compartments disposed between the anode and cathode compartments, wherein two or more ion-permeable barriers are ion-permeable isoelectric barriers each having a different pl value.

15. The method according to claim 14 wherein three or more of the ion-permeable barriers are ion-permeable isoelectric barriers each having a different pl value.

16. The method according to claim 14 wherein all the ion-permeable barriers are ion-permeable isoelectric barriers each having a different pl value.

17. The method according to any one of claims 9 to 16 wherein a first isoelectric buffer and a second isoelectric buffer each having a different pl value are provided to at least two of the sample compartments or the anode compartment or the cathode compartment.

18. The method according to any one of claims 1 to 17 wherein the ion-permeable barriers are selected from the group consisting of immiscible liquids, porous solids, non-ionic membranes, membranes, isoelectric membranes, hydrogel membranes, gels, and hydrogels.

19. The method according to 18 wherein the hydrogel membranes are polyether sulfone-based, poly(vinyl) alcohol-based or polyacrylamide-based.

20. The method according to claim 18 wherein the non-ionic membranes are supported membranes selected from the group consisting of cross-linked polyacrylamide and poly(vinyl) alcohol supported on glass fiber, filter paper, polymeric mesh, and paper.

21. The method according to claim 18 wherein the ion-permeable barriers are porous frits selected from the group consisting of glass frits, ceramic frits, and polymeric frits.

22. The method according to any one of claims 18 to 21 wherein the ion-permeable barriers substantially restrict the passage of molecules having a size or hydrated ion radius greater than a predetermined size.

23. The method according to any one of claims 1 to 17 wherein the ion-permeable isoelectric barriers are selected from the group consisting of isoelectric porous solids, isoelectric membranes, and isoelectric gels.

24. The method according to claim 23 wherein the ion-permeable isoelectric barriers are isoelectric membranes.

25. The method according to any one of claims 1 to 24 wherein there is substantially no convective mixing between the compartments.

26. The method according to any one of claims 1 to 25 wherein the isoelectric buffer is provided to at least one of a sample compartment, the anode compartment and the cathode compartment

27. The method according to claim 26 wherein the pl value of the isoelectric buffer differs by at least 0.001 pH units from the pl value of the ampholytic sample component.

28. The method according to any one of claims 1 to 27 wherein the absolute value of the difference between the pl value and the pKa value closest to the pl value of the isoelectric buffer is less than about 1.5.

29. The method according to any one of claims 1 to 28 wherein the isoelectric buffer is selected from the group consisting of iminodiacetic acid, N-methylimino diacetic acid, aspartic acid, glutamic acid, glycyl-aspartic acid, m-aminobenzoic acid, histidyl-glycine, histidyl-histidine, histidine, 1,2-diaminopropionic acid, omithine, lysine, lysil-lysine, and arginine.

30. The method according to claim 29 wherein the isoelectric buffer is glutamic acid or lysine.

31. The method according claim 17, wherein the first isoelectric buffer is glutamic acid and the second isoelectric buffer is lysine.

32. The method according to any one of claims 1 to 31 further comprising providing a non-ionic solubilizing agent.

33. The method according to claim 32 wherein the solubilizing agent is a non-ionic detergent.

34. The method according to any one of claims 1 to 33 wherein the ampholytic sample component is selected from the group consisting of proteins, polypeptides, oligopeptides, amino phenols, amino phosphonic acids, and amino acids.

35. The method according to claim 34, wherein the ampholytic sample component is a protein.

## Patentansprüche

1. Verfahren zum Abtrennen eines ampholytischen Bestandteiles durch Elektrophorese, wobei das Verfahren umfasst:
Anordnen einer Probe, die einen ampholytischen Bestandteil mit einem pI-Wert enthält, in einem Elektrophoresetrennsystem umfassend einen Anolyten mit einem pH und einen Katolyten mit einem pH, wobei der pH des Katolyten höher ist als der pH des Anolyten, eine oder mehrere Ionen-permeable Barrieren, die zwischen dem Anolyten und dem Katolyten angeordnet sind, wobei wenigstens eine der Barrieren eine isoelektrische Barriere mit einem pI-Wert ist, der höher ist als der pH des Anolyten und niedriger ist als der pH des Katolyten;
Bereitstellen eines isoelektrischen Puffers mit einem pI-Wert, der höher ist als der pH des Anolyten und niedriger ist als der pH des Katolyten und verschieden ist von dem pI-Wert des ampholytischen Probenbestandteiles und verschieden ist von dem pI-Wert einer Ionen-permeablen isoelektrischen Barriere; und
Exponieren der Probe gegenüber einem elektrischen Potential, um den ampholytischen Probenbestandteil in einem nicht-isoelektrischen Zustand in Gegenwart des isoelektrischen Puffers in dem Elektrophoresesystem einzufangen.

2. Verfahren nach Anspruch 2, wobei das Elektrophoresetrennsystem ein Anodenkompartiment, das eine Anode enthält und ausgeführt ist, um den Anolyten aufzunehmen, ein Kathodenkompartiment, das eine Kathode enthält und ausgeführt ist, um den Katolyten aufzunehmen, und eine Ionen-permeable Barriere in der Form einer Ionen-permeablen isoelektrischen Barriere mit einem pI-Wert, die zwischen dem Anodenkompartiment und dem Kathodenkompartiment angeordnet ist, umfasst.

3. Verfahren nach Anspruch 2, wobei das Elektrophoresetrennsystem weiter eine erste und eine zweite Ionen-permeable Barriere umfasst, die zwischen dem Anodenkompartiment und dem Kathodenkompartiment angeordnet sind und die ein erstes Trennkompartiment zwischen dem Anodenkompartiment und dem Kathodenkompartiment ausbilden.

4. Verfahren nach Anspruch 3, wobei die erste und zweite Ionen-permeable Barriere Ionen-permeable isoelektrische Barrieren mit jeweils einem definierten aber unterschiedlichen pI sind.

5. Verfahren nach Anspruch 3 oder 4, wobei die Probe in entweder dem Probenkompartiment, dem Anodenkompartiment oder dem Kathodenkompartiment bereitgestellt wird.

6. Verfahren nach Anspruch 5, wobei die Probe in dem Probenkompartiment bereitgestellt wird und der ampholytische Bestandteil in einem nicht-isoelektrischen Zustand in dem Probenkompartiment abgetrennt wird.

7. Verfahren nach Anspruch 5, wobei der ampholytische Bestandteil in einem nicht-isoelektrischen Zustand in dem Probenkompartiment oder dem Kathodenkompartiment oder Anodenkompartiment erhalten wird.

8. Verfahren nach Anspruch 3 oder 4, wobei die Probe zwei oder mehr ampholytische Bestandteile enthält, die in nicht-isoelektrischen Zuständen in dem Probenkompartiment oder dem Kathodenkompartiment oder dem Anodenkompartiment oder jeweils zwei oder mehr von dem Probenkompartiment, dem Anodenkompartiment oder dem Kathodenkompartiment erhalten werden.

9. Verfahren nach Anspruch 3, wobei das Elektrophoresetrennsystem weiter eine weitere Ionen-permeable Barriere umfasst, die zwischen dem Anodenkompartiment und dem Kathodenkompartiment angeordnet ist und die ein zweites Trennkompartiment benachbart dem ersten Trennkompartiment ausbildet, wobei das System eine erste Ionen-permeable isoelektrische Barriere mit einem ersten pI-Wert und eine zweite Ionen-permeable isoelektrische Barriere mit einem zweiten pI-Wert aufweist, der von dem ersten pI-Wert der ersten Ionen-permeablen isoelektrischen Barriere verschieden ist.

10. Verfahren nach Anspruch 9, wobei die Ionen-permeablen Barrieren alle isoelektrische Barrieren mit jeweils einem definierten aber unterschiedlichen pI sind.

11. Verfahren nach Anspruch 9 oder 10, wobei die Probe in dem ersten Probenkompartiment, dem zweiten Probenkompartiment, dem Anodenkompartiment oder dem Kathodenkompartiment oder sowohl dem ersten als auch dem zweiten Probenkompartiment bereitgestellt wird.

12. Verfahren nach Anspruch 11, wobei der ampholytische Bestandteil in einem nicht-isoelektrischen Zustand in dem ersten Probenkompartiment oder in dem zweiten Probenkompartiment oder in dem Anodenkompartiment oder dem Kathodenkompartiment erhalten wird.

13. Verfahren nach Anspruch 9 oder 10, wobei die Probe zwei oder mehr ampholytische Bestandteile enthält, die in einem nicht-isoelektrischen Zustand in dem ersten Probenkompartiment oder in dem zweiten Probenkompartiment, oder in sowohl dem ersten Probenkompartiment als auch in dem zweiten Probenkompartiment erhalten werden.

14. Verfahren nach Anspruch 2, wobei das Elektrophoresetrennsystem weiter eine Vielzahl von Ionen-permeablen Barrieren umfasst, die zwischen dem Anodenkompartiment und dem Kathodenkompartiment angeordnet sind und die eine Vielzahl von Trennkompartimenten ausbilden, die zwischen den Anoden- und Kathodenkompartimenten angeordnet sind, wobei zwei oder mehr Ionen-permeable Barrieren Ionen-permeable isoelektrische Barrieren mit jeweils einem unterschiedlichen pI-Wert sind.

15. Verfahren nach Anspruch 14, wobei drei oder mehr der Ionen-permeablen Barrieren Ionen-permeable isoelektrische Barrieren mit jeweils einem unterschiedlichen pI-Wert sind.

16. Verfahren nach Anspruch 14, wobei alle Ionen-permeablen Barrieren Ionen-permeable isoelektrische Barrieren mit jeweils einem unterschiedlichen pI-Wert sind.

17. Verfahren nach einem der Ansprüche 9 bis 16, wobei ein erster isoelektrischer Puffer und ein zweiter isoelektrischer Puffer mit jeweils einem unterschiedlichen pI-Wert in wenigstens jeweils zwei von den Probenkompartimenten oder dem Anodenkompartiment oder dem Kathodenkompartiment bereitgestellt werden.

18. Verfahren nach einem der Ansprüche 1 bis 17, wobei die Ionen-permeablen Barrieren ausgewählt sind aus der Gruppe bestehend aus nicht mischbaren Flüssigkeiten, porösen Feststoffen, nicht-ionischen Membranen, Membranen, isoelektrischen Membranen, Hydrogelmembranen, Gelen und Hydrogelen.

19. Verfahren nach Anspruch 18, wobei die Hydrogelmembranen Polyethersulfon-basiert, Poly(vinyl)alkohol-basiert oder Polyacrylamid-basiert sind.

20. Verfahren nach Anspruch 18, wobei die nicht-ionischen Membranen gestützte Membranen sind, die ausgewählt sind aus der Gruppe bestehend aus quervernetztem Polyacrylamid und Poly(vinyl)alkohol, gestützt auf Glasfaser, Filterpapier, Polymergitter und Papier.

21. Verfahren nach Anspruch 18, wobei die Ionen-permeablen Barrieren poröse Fritten sind, die ausgewählt sind aus der Gruppe bestehend aus Glasfritten, Keramikfritten und Polymerfritten.

22. Verfahren nach einem der Ansprüche 18 bis 21, wobei die Ionen-permeablen Barrieren im wesentlichen den Durchgang von Molekülen mit einer Größe oder einem hydratisierten Ionenradius beschränken, die/der größer ist als eine festgelegte Größe.

23. Verfahren nach einem der Ansprüche 1 bis 17, wobei die Ionen-permeablen isoelektrischen Barrieren ausgewählt sind aus der Gruppe bestehend aus isoelektrischen porösen Feststoffen, isoelektrischen Membranen und isoelektrischen Gelen.

24. Verfahren nach Anspruch 23, wobei die Ionen-permeablen isoelektrischen Barrieren isoelektrische Membranen sind.

25. Verfahren nach einem der Ansprüche 1 bis 24, wobei im wesentlichen kein Konvektionsmischung zwischen den Kompartimenten auftritt.

26. Verfahren nach einem der Ansprüche 1 bis 25, wobei der isoelektrische Puffer in wenigstens einem von dem Probenkompartiment, dem Anodenkompartiment und dem Kathodenkompartiment bereitgestellt wird.

27. Verfahren nach Anspruch 26, wobei sich der pI-Wert des isoelektrischen Puffers um wenigstens 0,001 pH-Einheiten von dem pI-Wert des ampholytischen Probenbestandteils unterscheidet.

28. Verfahren nach einem der Ansprüche 1 bis 27, wobei der Absolutwert des Unterschiedes zwischen dem pI-Wert und dem pKa-Wert, der zu dem pI-Wert des isoelektrischen Puffers am nächsten ist, weniger als etwa 1,5 beträgt.

29. Verfahren nach einem der Ansprüche 1 bis 28, wobei der isoelektrische Puffer ausgewählt ist aus der Gruppe bestehend aus Iminoacetessigsäure, N-Methyliminoacetessigsäure, Asparaginsäure, Glutaminsäure, Glycyl-Asparaginsäure, m-Aminobenzoesäure, Histidyl-Glycin, Histidyl-Histidin, Histidin, 1,2-Diaminopropionsäure, Omithine, Lysin, Lysil-Lysin, und Arginin.

30. Verfahren nach Anspruch 29, wobei der isoelektrische Puffer Glutaminsäure oder Lysin ist.

31. Verfahren nach Anspruch 17, wobei der isoelektrische Puffer Glutaminsäure ist und der zweite isoelektrische Puffer Lysin ist.

32. Verfahren nach einem der Ansprüche 1 bis 31, weiter umfassend Bereitstellen eines nicht-ionischen lösungsvermittelnden Agens.

33. Verfahren nach Anspruch 32, wobei das lösungsvermittelnde Agens ein nichtionisches Detergens ist.

34. Verfahren nach einem der Ansprüche 1 bis 33, wobei der ampholytische Probenbestandteil ausgewählt ist aus der Gruppe bestehend aus Proteinen, Polypeptiden, Oligopeptiden, Aminophenolen, Aminophosphonsäuren und Aminosäuren.

35. Verfahren nach Anspruch 34, wobei der ampholytische Probenbestandteil ein Protein ist.

## Revendications

1. Procédé de séparation d'un constituant ampholytique par électrophorèse, le procédé consistant:
à placer un échantillon contenant un constituant ampholytique ayant une valeur pl dans un système de séparation par électrophorèse qui comprend un anolyte ayant un pH et un catholyte ayant un pH, le pH du catholyte étant supérieur au pH de l'anolyte, une ou plusieurs barrières perméables aux ions étant disposées entre l'anolyte et le catholyte, dans lequel au moins l'une des barrières est une barrière isoélectrique dont la valeur pl est supérieure au pH de l'anolyte et inférieure au pH du catholyte;
à fournir un tampon isoélectrique ayant une valeur pl supérieure au pH de l'anolyte et inférieure au pH du catholyte, et qui est différente de la valeur pl du constituant de l'échantillon ampholytique et différente de la valeur pl d'une barrière isoélectrique perméable aux ions; et
à exposer l'échantillon à un potentiel électrique de manière à capter le constituant échantillon ampholytique à l'état non isoélectrique en présence du tampon isoélectrique dans le système d'électrophorèse.

2. Procédé selon la revendication 1, dans lequel le système de séparation par électrophorèse comporte un compartiment anodique renfermant une anode et adapté pour recevoir l'anolyte, un compartiment cathodique renfermant une cathode et adapté pour recevoir le catholyte, ainsi qu'une barrière perméable aux ions qui se présente sous forme de barrière isoélectrique perméable aux ions ayant une valeur pl, qui est disposée entre le compartiment anodique et le compartiment cathodique.

3. Procédé selon la revendication 2, dans lequel le système de séparation par électrophorèse comporte par ailleurs une première et une deuxième barrières perméables aux ions disposées entre le compartiment anodique et le compartiment cathodique, et formant ainsi un premier compartiment de séparation entre le compartiment anodique et le compartiment cathodique.

4. Procédé selon la revendication 3, dans lequel les première et deuxième barrières perméables aux ions sont des barrières isoélectriques perméables aux ions ayant chacune une valeur pl définie mais différente.

5. Procédé selon la revendication 3 ou la revendication 4, dans lequel l'échantillon est envoyé dans au moins l'un des compartiments suivants: le compartiment porte-échantillon, le compartiment anodique et le compartiment cathodique.

6. Procédé selon la revendication 5, dans lequel l'échantillon est envoyé dans le compartiment porte-échantillon, et le constituant ampholytique est séparé à l'état non isoélectrique à l'intérieur du compartiment porte-échantillon.

7. Procédé selon la revendication 5, dans lequel le constituant ampholytique s'obtient à l'état non isoélectrique dans le compartiment porte-échantillon, ou dans le compartiment cathodique, ou dans le compartiment anodique.

8. Procédé selon la revendication 3 ou la revendication 4, dans lequel l'échantillon contient deux constituants ampholytiques ou davantage, qui s'obtiennent à l'état non isoélectrique dans le compartiment porte-échantillon, ou dans le le compartiment cathodique, ou dans le compartiment anodique, ou bien dans deux compartiments ou davantage parmi le compartiment porte-échantillon, le compartiment anodique, et le compartiment cathodique.

9. Procédé selon la revendication 3, dans lequel le système de séparation par électrophorèse comporte par ailleurs une autre barrière perméable aux ions disposée entre le compartiment anodique et le compartiment cathodique, formant un deuxième compartiment de séparation à côté du premier compartiment de séparation, le système comportant une première barrière isoélectrique perméable aux ions ayant une première valeur pl, et une deuxième barrière isoélectrique perméable aux ions ayant une deuxième valeur pl qui est différente de la première valeur pl de la première barrière isoélectrique perméable aux ions.

10. Procédé selon la revendication 9, dans lequel les barrières perméables aux ions sont toutes des barrières isoélectriques ayant chacune une valeur pl définie mais différente.

11. Procédé selon la revendication 9 ou la revendication 10, dans lequel l'échantillon est envoyé dans le premier compartiment porte-échantillon, dans le deuxième compartiment porte-échantillon, dans le compartiment anodique ou dans le compartiment cathodique, ou bien dans les premier et deuxième compartiments porte-échantillon.

12. Procédé selon la revendication 11, dans lequel le constituant ampholytique s'obtient à l'état non isoélectrique dans le premier compartiment porte-échantillon ou dans le deuxième compartiment porte-échantillon, ou dans le compartiment anodique, ou dans le compartiment cathodique.

13. Procédé selon la revendication 9 ou la revendication 10, dans lequel l'échantillon contient deux constituants ampholytiques ou davantage qui s'obtiennent à l'état non isoélectrique dans le premier compartiment porte-échantillon ou dans le deuxième compartiment porte-échantillon, ou bien dans le premier compartiment porte-échantillon ainsi que dans le deuxième compartiment porte-échantillon.

14. Procédé selon la revendication 2, dans lequel le système de séparation par électrophorèse comporte par ailleurs une pluralité de barrières perméables aux ions disposées entre le compartiment anodique et le compartiment cathodique formant ainsi une pluralité de compartiments de séparation entre les compartiments anodique et cathodique, dans lequel deux barrières perméables aux ions ou davantage sont des barrières isoélectriques perméables aux ions ayant chacune une valeur pl différente.

15. Procédé selon la revendication 14, dans lequel trois barrières perméables aux ions ou davantage sont des barrières isoélectriques perméables aux ions ayant chacune une valeur pl différente.

16. Procédé selon la revendication 14, dans lequel toutes les barrières perméables aux ions sont des barrières isoélectriques perméables aux ions ayant chacune une valeur pl différente.

17. Procédé selon l'une quelconque des revendications 9 à 16, **caractérisé en ce qu'**un premier tampon isoélectrique et un deuxième tampon isoélectrique, ayant chacun une valeur pl différente, sont envoyés dans au moins deux des compartiments porte-échantillon ou dans le compartiment anodique ou le compartiment cathodique.

18. Procédé selon l'une quelconque des revendications 1 à 17, dans lequel les barrières perméables aux ions sont sélectionnées dans le groupe comprenant des liquides non miscibles, des solides poreux, des membranes non ioniques, des membranes, des membranes isoélectriques, des membranes à base d'hydrogels, des gels et des hydrogels.

19. Procédé selon la revendication 18, dans lequel les membranes à base d'hydrogels sont à base de sulfone de polyéther, à base de polyalcools/d'alcools polyvinyliques, ou à base de polyacrylamide.

20. Procédé selon la revendication 18, dans lequel les membranes non ioniques sont des membranes sur support sélectionnées dans le groupe comprenant du polyacrylamide réticulé et du polyalcool/de l'alcool polyvinylique porté sur de la fibre de verre, du papier filtre, de la maille polymérique, et du papier.

21. Procédé selon la revendication 18, dans lequel les barrières perméables aux ions sont des frittes poreuses sélectionnées dans le groupe comprenant des frittes de verre, des frittes de céramique, et des frittes polymériques.

22. Procédé selon l'une quelconque des revendications 18 à 21, dans lequel les barrières perméables aux ions limitent en grande partie le passage des molécules dont la taille ou le rayon des ions hydratés sont supérieurs à une taille prédéterminée.

23. Procédé selon l'une quelconque des revendications 1 à 17, dans lequel les barrières isoélectriques perméables aux ions sont sélectionnées dans le groupe comprenant des solides poreux isoélectriques, des membranes isoélectriques, et des gels isoélectriques.

24. Procédé selon la revendication 23, dans lequel les barrières isoélectriques perméables aux ions sont des membranes isoélectriques.

25. Procédé selon l'une quelconque des revendications 1 à 24, **caractérisé en ce qu'**il ne se produit essentiellement aucun mélange convectif entre les compartiments.

26. Procédé selon l'une quelconque des revendications 1 à 25, dans lequel le tampon isoélectrique est envoyé dans au moins l'un des compartiments suivants: le compartiment porte-échantillon, le compartiment anodique et le compartiment cathodique.

27. Procédé selon la revendication 26, dans lequel la valeur pl du tampon isoélectrique s'écarte d'au moins 0,001 unité de pH par rapport à la valeur pl du constituant de l'échantillon ampholytique.

28. Procédé selon l'une quelconque des revendications 1 à 27, dans lequel la valeur absolue de la différence entre la valeur pl et la valeur pKa qui est la plus proche de la valeur pl du tampon isoélectrique est inférieure à 1,5 environ.

29. Procédé selon l'une quelconque des revendications 1 à 28, dans lequel le tampon isoélectrique est sélectionné dans le groupe comprenant l'acide iminodiacétique, l'acide N-méthylimino diacétique, l'acide aspartique, l'acide glutamique, l'acide glycyl-aspartique, l'acide m-aminobenzoïque, l'histidyl-glycine, l'histidyl-histidine, l'histidine, l'acide 1,2-diaminopropionique, l'omithine, la lysine, la lysil-lysine, et l'arginine.

30. Procédé selon la revendication 29, dans lequel le tampon isoélectrique est l'acide glutamique ou la lysine.

31. Procédé selon la revendication 17, dans lequel le premier tampon isoélectrique est l'acide glutamique et le deuxième tampon isoélectrique est la lysine.

32. Procédé selon l'une quelconque des revendications 1 à 31, fournissant par ailleurs un agent solubilisant non ionique.

33. Procédé selon la revendication 32, dans lequel l'agent solubilisant est un détergent non ionique.

34. Procédé selon l'une quelconque des revendications 1 à 33, dans lequel le constituant de l'échantillon ampholytique est sélectionné dans le groupe comprenant les protéines, les polypeptides, les oligopeptides, les phénols aminés, les acides amino-phosphoniques, et les acide aminés.

35. Procédé selon la revendication 34, dans lequel le constituant de l'échantillon ampholytique est une protéine
